# EUROPEAN PATENT APPLICATION

(11) **EP 2 404 569 A2**
(43) Date of publication of application: **11.01.2012**
(21) Application number: 10748949.4
(22) Date of filing: 03.03.2010
(51) Int. Cl.: A61C 17/10, A61B 1/24

(54) **TIPS FOR PROTECTING THE TONGUE, BITE BLOCK, AND INTRAORAL ILLUMINATION DEVICE HAVING A SALIVA-SUCKING FUNCTION**

(30) Priority: 06.03.2009 KR 20090019328
(71) Applicant: Dentozone Co., Ltd., Geumcheon-Gu, Seoul 153-801 (KR)
(72) Inventor: KOO, Cha Hyoung, Seoul 153-013 (KR)
(74) Representative: Beck & Rössig European Patent Attorneys
(86) International application number: PCT/KR2010/001322
(87) International publication number: WO 2010/101407

(57) **Abstract**

A bite block, an illumination device having a saliva suction function, and a TIPS for protecting the tongue of a patient are disclosed. The present invention guides the tongue of the patient such that when dental treatment is performed, the tongue of the patient can be safely and comfortably protected. Furthermore, saliva collected around the throat of the patient can be rapidly removed so that the patient feels little foreign-body like sensations. In addition, the TIPS can isolate the tongue from one side of the mouth cavity to be treated so as to ensure space for treatment. Moreover, the present invention can illuminate the entirety of the mouth cavity of the patient to enable a doctor to conveniently conduct the dental treatment.

## Description

### [Technical Field]

The present invention relates, in general, to a bite block, an illumination device having a saliva suction function and to TIPSs (twist isolated plates of suction) for protecting the tongues of patients, and more particularly, to a bite block, an illumination device having a saliva suction function, and to a TIPS for protecting the tongue of a patient, in which when dental treatment is performed, the tongue of the patient can be safely and comfortably protected, and saliva collected around the throat of the patient can be rapidly removed so that the patient hardly senses the presence of a foreign-body, and which can isolate the tongue from one side of the mouth cavity to be treated so as to ensure space for treatment, and which can illuminate the entirety of the mouth cavity of the patient to enable a doctor to conveniently conduct the dental treatment.

### [Background Art]

Generally, in dental treatment, an external light source illuminates the mouth cavity of a patient to enable a doctor and a nurse to easily and clearly observe the mouth cavity.

However, it is difficult even for a skilled doctor or nurse to clearly illuminate the mouth cavity of a patient using the external light source and simultaneously perform dental treatment. Due to the characteristic of the structure of the mouth cavity being typically narrow and deep, the external light source cannot clearly illuminate the entirety of the mouth cavity.

In an effort to overcome the above-mentioned problems, an intraoral illumination device which is inserted into the mouth of a patient to illuminate the mouth cavity and suck saliva collected in the mouth was recently proposed in Korean Patent Registration No. 10-0654392.

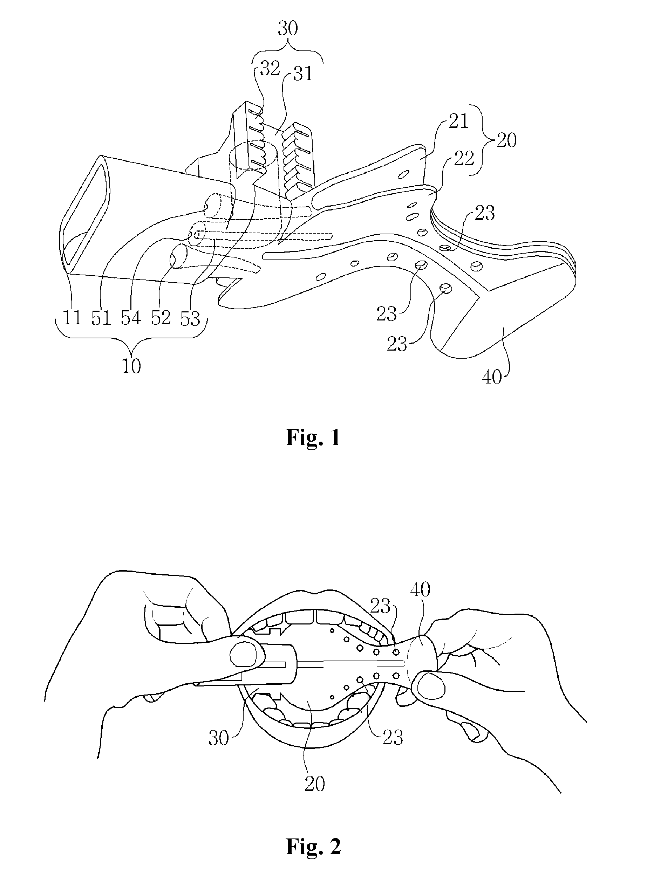

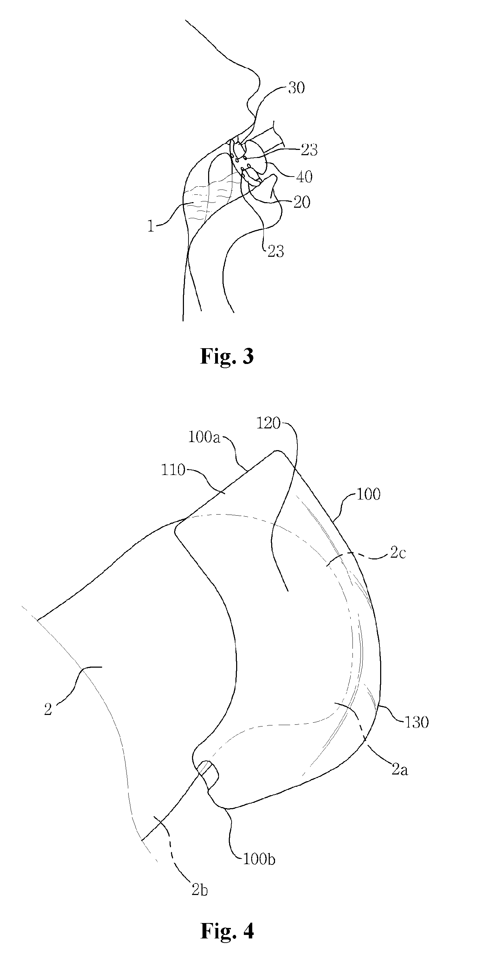

FIG. 1 is a view showing the conventional intraoral illumination device. FIG. 2 is a view showing an example of installing the conventional intraoral illumination device in the mouth of a patient. FIG. 3 is a view illustrating the saliva sucking operation of the conventional intraoral illumination device.

Referring to the drawings, the conventional intraoral illumination device includes a tube coupling part 10, a tongue protection part 20 and a bite block 30. A tube coupling hole 11 to which an optical fiber is coupled is formed on a first side of the tube coupling part 10. The tongue protection part 20 is provided on the central portion of the device and includes upper and lower blades 21 and 22 which press the tongue of the patient rearwards. The bite block 30 is provided between the tube coupling part 10 and the tongue protection part 20 so that the bite block 30 is easily held in the mouth in such a way that the patient bites the bite block 30 with his/her teeth.

In the conventional intraoral illumination device, the bite block 30 is placed between the upper and lower teeth in a first side of the mouth cavity of the patient and bitten, and the tongue protection part 20 is inserted into the mouth and presses the tongue of the patient such that the tongue is pushed rearwards to provide space for a doctor to provide medical treatment. Also, the intraoral illumination device illuminates the mouth cavity.

To illuminate the mouth cavity, the optical fiber is inserted into the optical fiber coupling hole 53 of the tube coupling part 10.

Because the optical fiber emits light in the mouth, it can evenly illuminate the entirety of the mouth cavity.

Furthermore, block protrusions 32 and a block depression 31 are formed in the bite block 30 to prevent the intraoral illumination device from slipping out of between the teeth of the patient. A cheek support part 40 is held between the upper and lower teeth positioned at a second side of mouth such that the intraoral illumination device can be more reliably supported in the mouth of the patient.

In addition, a first suction hole 51, a second suction hole 52 and a block hole 54 are formed in the tube coupling part 10. Small suction holes 23 are formed through the tongue protection part 20. The intraoral illumination device sucks saliva 1 from the mouth cavity through the first suction hole 51, the second suction hole 52, the block hole 54 and the small suction holes 23 and then discharges the saliva 1 out of the mouth.

The small suction holes 23 can suck saliva at several portions from the mouth cavity, but they are not directly connected to the suction holes 51, 52 and 54, resulting in a comparatively large loss of suction force.

In addition, in the conventional intraoral illumination device, because the tongue protection part 20 pushes the tongue of the patient rearwards, the patient may feel excessive pressure on the tongue. Moreover, only when saliva 1 is charged from the throat to the tip of the tongue can the saliva 1 be sucked out of the mouth through the small suction holes 23. Thus, there is a disadvantage in that the patient senses the saliva 1 as if it were a foreign body.

Furthermore, in the conventional intraoral illumination device, the tube coupling part 10, the tongue protection part 20 and the bite block 30 are integrally formed into a single body, thus increasing the entire size of the device. The large size of the device may cause the patient apprehension. In addition, due to its large size, it becomes difficult to wash the device.

### [Disclosure]

### [Technical Problem]

The inventors of the present invention completed the present invention resulting from efforts to develop an intraoral illumination device which can reliably protect the tongue of a patient when dental treatment is performed, and can rapidly remove saliva out of the mouth of the patient, and can be effectively washed, and can clearly illuminate the entirety of the mouth cavity.

Accordingly, an object of the present invention is to provide a TIPS for protecting the tongue of a patient, a bite block and an illumination device which can isolate the tongue from a side of the mouth cavity to be treated without pressing the tongue, so that when dental treatment is performed, the tongue can be reliably protected, and a space for treatment can be sufficiently ensured in the first side of the mouth cavity to be treated, and which can evenly illuminate the mouth cavity.

Another object of the present invention is to provide a TIPS, a bite block and an illumination device which can quickly suck saliva accumulating and rising up from the throat without lowering the force of suction and then discharge the saliva out of the mouth, thus minimizing saliva being sensed as if it were a foreign-body.

A further object of the present invention is to provide a TIPS, a bite block and an illumination device in which the bite block is bitten between the first side of the mouth cavity to open the mouth, and a light source is inserted into the bite block and thus is able to directly and effectively illuminate the mouth cavity.

Yet another object of the present invention is to provide a TIPS, a bite block and an illumination device which can be separated from each other, thus facilitating its being washed.

Still another object of the present invention is to provide a TIPS, a bite block and an illumination device which can sterilize the mouth cavity when the treatment is being performed and can record images of the treatment process and provides the images to a doctor or the patient.

The objects of the present invention are not limited to the above-mentioned objects, and other unmentioned objects will be clearly interpreted by those skilled in the art from the following description.

### [Technical Solution]

In order to accomplish the above objects, in an aspect, the present invention provides a TIPS (twist isolated plate of suction) for protecting a tongue of a patient when a dental treatment is performed, the TIPS being removably coupled to a bite block bitten between upper and lower teeth in a first side of a mouth of the patient to open the mouth of the patient. The TIPS has a first side coupled to the bite block, and a second side covering a first side surface of the tongue and extending towards a second-side tongue root in the mouth.

In an embodiment, the TIPS may include a block coupling part coupled to the bite block, a TIPS body extending from the block coupling part and being curved towards the second-side tongue root, and a tongue protection part extending downwards from an edge of the TIPS body to cover the first side surface of the tongue.

The tongue protection part may cover a tip of the tongue and isolate the tongue from teeth positioned in a second side of the mouth of the patient.

The second side of the TIPS may extend from the first side of the TIPS along an imaginary extension axis. The second side of the TIPS may be twisted around the imaginary extension axis at a predetermined angle to surround the first side surface of the tongue.

Furthermore, a saliva suction tube may be formed through the first side of the TIPS and the second side of the TIPS to suck saliva collected between a throat of the patient and the second-side tongue root.

The saliva suction tube may comprise a plurality of saliva suction tubes.

In another aspect, the present invention provides a bite block, including a block body bitten between upper and lower teeth in a first side of a mouth of a patient to open a mouth of the patient, a light source insert hole formed in a first side of the block body so that an external light source is inserted into the light source insert hole, a TIPS coupling hole formed in the block body at a position facing a center of the mouth so that a TIPS is coupled to the TIPS coupling hole, and a saliva transfer tube formed through the first side of the block body at a position spaced apart from the light source, the saliva transfer tube communicating with the TIPS coupling hole and being connected to an external suction tube so that saliva drawn into the TIPS coupling hole is transferred to the external suction tube through the saliva transfer tube.

In an embodiment, the TIPS coupling hole may comprise a plurality of TIPS coupling holes spaced apart from each other. The saliva transfer tube may comprise a plurality of saliva transfer tubes spaced apart from each other, the saliva transfer tubes respectively communicating with the TIPS coupling holes.

The TIPS coupling holes may comprise two TIPS coupling holes including a first TIPS coupling hole and a second TIPS coupling hole. The saliva transfer tubes may comprise a first saliva transfer tube and a second saliva transfer tube which are respectively connected to the first TIPS coupling hole and the second TIPS coupling hole.

The TIPS coupling hole may comprise a plurality of TIPS coupling holes spaced apart from each other. The saliva transfer tube may branch out into two parts connected to the respective TIPS coupling holes.

The TIPS coupling holes may comprise two TIPS coupling holes including a first TIPS coupling hole and a second TIPS coupling hole which are spaced apart from each other.

Furthermore, bite depressions may be respectively formed in upper and lower portions of the block body which are bitten between the upper and lower teeth of the patient.

In addition, an anti-slip uneven surface may be formed in each of the bite depressions to prevent the block body from slipping relative to the upper or lower teeth of the patient.

The anti-slip uneven surface may be formed in a bottom of the bite depression.

The block body may comprise a transparent block body allowing light emitted from the light source to be transmitted through the block body.

In a further aspect, the present invention provides an intraoral illumination device, including a handpiece comprising a light source provided on a first end thereof, a bite block coupled to the first end of the handpiece, the bite block transmitting light of the light source into a mouth of a patient and being bitten between upper and lower teeth in a first side of the mouth to open the mouth, and a TIPS for protecting a tongue of the patient, the TIPS having a first side coupled to the bite block, and a second side covering a first side surface of the tongue of the patient and extending towards a second-side tongue root in the mouth.

In an embodiment, the TIPS may include a block coupling part coupled to the bite block, a TIPS body extending from the block coupling part and being curved towards the second-side tongue root, and a tongue protection part extending downwards from an edge of the TIPS body to cover the first side surface of the tongue.

The tongue protection part may cover a tip of the tongue and isolate the tongue from teeth positioned in a second side of the mouth of the patient.

The second side of the TIPS may extend from the first side of the TIPS along an imaginary extension axis. The second side of the TIPS may be twisted around the imaginary extension axis at a predetermined angle to surround the first side surface of the tongue.

Furthermore, a saliva suction tube may be formed through the first side of the TIPS and the second side of the TIPS to suck saliva collected between a throat of the patient and the second-side tongue root.

The saliva suction tube may comprise a plurality of saliva suction tubes.

The bite block may have a light source insert hole formed in a first side of the block body so that the light source is inserted into the light source insert hole, and a TIPS coupling hole formed in the block body at a position facing a center of the mouth so that the TIPS is coupled to the TIPS coupling hole.

The handpiece may further include a suction tube provided through the first end and a second of the handpiece, the suction tube being connected to an external suction device. The bite block may further include a saliva transfer tube connected to the suction tube, the saliva transfer tube communicating with the TIPS coupling hole so that saliva drawn into the TIPS coupling hole is transferred to the suction tube through the saliva transfer tube.

The TIPS coupling hole may comprise a plurality of TIPS coupling holes spaced apart from each other. The saliva transfer tube may comprise a plurality of saliva transfer tubes spaced apart from each other, the saliva transfer tubes respectively communicating with the TIPS coupling holes.

The suction tube may comprise a plurality of suction tubes. The saliva transfer tubes may be respectively connected to the suction tubes.

The suction tubes may comprise two suction tubes including a first suction tube and a second suction tube. The TIPS coupling holes may comprise two TIPS coupling holes including a first TIPS coupling hole and a second TIPS coupling hole. The saliva transfer tubes may comprise a first saliva transfer tube and a second saliva transfer tube which are respectively connected to the first suction tube and the second suction tube, the first and second saliva transfer tubes respectively communicating with the first TIPS coupling hole and the second TIPS coupling hole.

The TIPS coupling hole may comprise a plurality of TIPS coupling holes spaced apart from each other. The saliva transfer tube may branch out into a plurality of parts communicating with the respective TIPS coupling holes.

Furthermore, bite depressions may be respectively formed in upper and lower portions of the block body which are bitten between the upper and lower teeth of the patient.

In addition, an anti-slip uneven surface may be formed in each of the bite depressions to prevent the block body from slipping relative to the upper or lower teeth of the patient.

The anti-slip uneven surface may be formed in a bottom of the bite depression.

The handpiece may include a valve to open or close the suction tubes.

The valve may comprise a single valve provided on the suction tubes in the handpiece, the valve selectively opening or closing the suction valve.

The handpiece may further include a valve control lever to control operation of the valve.

The handpiece may further include a light source control switch provided on an outer surface of the handpiece to turn on/off the light source.

The light source control switch may comprise a touch switch.

The bite block may comprise a transparent bite block allowing light emitted from the light source to transmit through the bite block body.

The light source may comprise an LED.

The intraoral illumination device may further include an ultraviolet lamp provided at a predetermined position on the handpiece to apply ultraviolet rays to an interior of the mouth.

The ultraviolet lamp may be provided on the light source.

The intraoral illumination device may further include a camera provided at a predetermined position on the handpiece to record images of the mouth when treatment is performed.

The camera may be provided on the light source.

### [Advantageous Effects]

The present invention has the following effects.

First, a TIPS, a bite block and an illumination device according to the present invention cover a side surface of the tongue of a patient without pressing the tongue rearwards and isolates the tongue from a portion of the mouth cavity to be treated to protect the tongue. Thereby, when the patient receives the dental treatment, his/her tongue can be kept comfortable and safe.

Furthermore, in the present invention, the TIPS pushes the tongue of the patient in the direction opposite to a side of the mouth cavity to be treated and thus isolates the tongue therefrom. Thereby, space for the dental treatment can be sufficiently ensured, thus enabling a doctor to conveniently perform the dental treatment.

In addition, the TIPS extends towards a tongue root adjacent to the side of the mouth cavity to be treated. Therefore, the present invention can suck saliva collected between the throat of the patient and the tongue root without lowering the force of suction before saliva accumulates and rises up to the tip of the tongue. Thus, the patient feels little foreign-body like sensations attributable to saliva.

Moreover, the TIPS, the bite block and the illumination device can be separated from each other, thus facilitating washing thereof. Therefore, the TIPS, the bite block and the illumination device can be always kept clean, thereby preventing the secondary infection of patients.

Also, the present invention includes an ultraviolet lamp and thus is able to prevent bacteria infection which may be caused when treatment is performed.

Furthermore, the present invention can record images of treatment using a camera and provide the images to a doctor or the patient.

### [Description of Drawings]

FIG. 1 is a view showing a conventional intraoral illumination device;
FIG. 2 is a view showing an example of installation of the conventional intraoral illumination device in the mouth of a patient;
FIG. 3 is a view illustrating a saliva sucking operation of the conventional intraoral illumination device;
FIG. 4 is a plan view illustrating an embodiment of a TIPS for protecting the tongue of a patient, according to the present invention;
FIG. 5 is a bottom view illustrating the TIPS according to the present invention;
FIG. 6 is a view illustrating a process of sucking saliva using the TIPS according to the present invention;
FIG. 7 is a view illustrating an embodiment of an intraoral illumination device according to the present invention;
FIG. 8 is an exploded perspective view of the intraoral illumination device according to the present invention; and
FIG. 9 is a view showing installation of the intraoral illumination device of the present invention in the mouth of the patient.

Reference should now be made to the drawings, in which the same reference numerals are used throughout the different drawings to designate the same or similar components.

### <Description of the elements in the drawings>

| | | | |
|---|---|---|---|
| 100: | TIPS | 110: | block coupling part |
| 120: | TIPS body | 130: | tongue protection part |
| 140: | saliva suction tube | 200: | bite block |
| 210: | block body | 211: | light source insert hole |
| 212: | TIPS coupling hole | 213: | saliva transfer tube |
| 214: | bite depression | 215: | uneven surface |
| 300: | handpiece | 310: | light source |
| 320: | light source control switch | 330: | suction tube |
| 340: | valve | 350: | valve control lever |

### [Best Mode]

The terminology which is used in common will be used for the purpose of description and not of limitation. Furthermore, terms and words used by the applicant may be used for special cases. In this case, the meaning of the terms or words must be understood with due regard to the meaning expressed in the specification rather than taking into account only the basic meaning of the terms or words.

Hereinafter, the technical construction of the present invention will be described in detail with reference to preferred embodiments illustrated in the attached drawings.

The present invention may, however, be embodied in different forms and should not be construed as limited to the embodiments set forth herein. The same reference numeral is used to refer to like elements throughout.

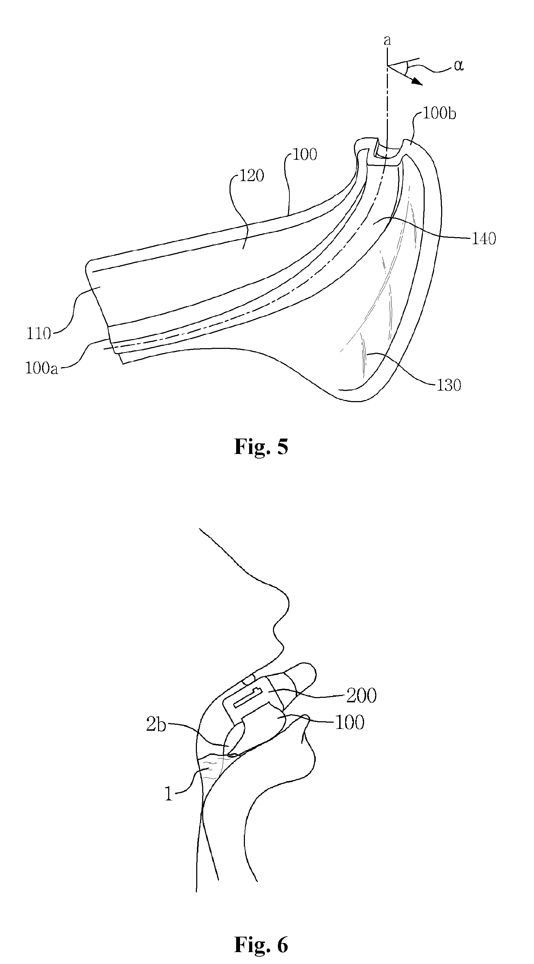

FIG. 4 is a plan view illustrating an embodiment of a TIPS for protecting the tongue of a patient, according to the present invention. FIG. 5 is a bottom view illustrating the TIPS according to the present invention. FIG. 6 is a view illustrating a process of sucking saliva using the TIPS according to the present invention.

Referring to the drawings, the TIPS 100 according to the embodiment of the present invention is removably coupled to a bite block 200 which is placed between the upper and lower teeth in a first side of the mouth of the patient to open the mouth and is then bitten. When dental treatment is performed, the TIPS 100 functions to protect the tongue 2 of the patient.

In detail, a first side 100a of the TIPS 100 is coupled to the bite block 200, and a second side 100b thereof covers a first side surface 2a of the tongue 2 and extends towards a second-side tongue root 2b in the mouth.

In other words, the TIPS 100 has a structure which is curved in a streamlined shape from the upper and lower teeth of the first side in the mouth towards the second-side tongue root 2b in the mouth.

Therefore, unlike the tongue protection part 20 of the conventional intraoral illumination device which presses the tongue downwards and thus pushes the tongue 2 rearwards in the mouth, the TIPS 100 of the present invention covers the first side surface 2a of the tongue 2 and biases the tongue 2 to the first side of the mouth, thus isolating the tongue 2 from a second side in the mouth. Thereby, space for treating teeth positioned in the second side of the mouth can be sufficiently ensured.

Therefore, the present invention enables a doctor to conveniently treat the interior of the mouth of the patient and assists the patient to comfortably and safely receive dental treatment without the tongue being pressed down upon.

Furthermore, in the conventional intraoral illumination device, the tongue protection part 20 pushes the tongue downwards and thus presses the tongue rearwards in the mouth. Thus, the patient cannot move the tongue 2. However, in the case of the TIPS 100 of the present invention, the patient can freely move the tongue 2 in the direction opposite to the first side surface 2a of the tongue 2 which is covered with the TIPS 100.

In addition, the TIPS 100 includes a block coupling part 110, a TIPS body 120 and a tongue protection part 130. The block coupling part 110 is provided at the first side 100a of the TIPS 100 and coupled to the bite block 200. The TIPS body 120 extends from the block coupling part 110 and is curved towards the second-side tongue root 2b. The tongue protection part 130 extends downwards from an edge of the TIPS body 120 to surround the first side surface 2a of the tongue 2.

The tongue protection part 130 can also cover a tip 2c of the tongue 2 and thus ensure space for treating the front teeth and the teeth positioned in the second side in the mouth of the patient.

Also, the TIPS 100 further includes a saliva suction tube 140 which is provided through the first side 100a and the second side 100b of the TIPS 100 to suck saliva 1, collected around the second-side tongue root 2b, out of the mouth.

The saliva suction tube 140 may comprise a plurality of saliva suction tubes.

Furthermore, the saliva suction tube 140 transfers saliva 1 from the second side 100b of the TIPS 100 to the first side 100a of the TIPS 100.

That is, as shown in FIG. 6, the TIPS 100 according to the present invention can immediately suck saliva 1, collected around the second-side tongue root 2b, out of the mouth through the saliva suction tube 140.

Therefore, compared to the saliva suction operation of the conventional intraoral illumination device which uses the small suction holes 23, the present invention reduce the drop in the force of suction because the saliva suction tube 140 can directly suck saliva 1. Furthermore, the saliva suction tube 140 can rapidly suck saliva 1 from around the tongue root, before the saliva 1 accumulates and rises up to the front portion of the tongue 2. Hence, the patient feels only a slight foreign-body like sensation attributable to saliva.

In the embodiment, the second side 110b of the TIPS 100 which extends from the first side 100a may be twisted around an imaginary extension axis a at a predetermined angle α to more reliably surround the first side surface 2a of the tongue 2.

Thereby, the TIPS 100 can reliably cover and protect even the lower portion of the first side surface 2a of the tongue 2.

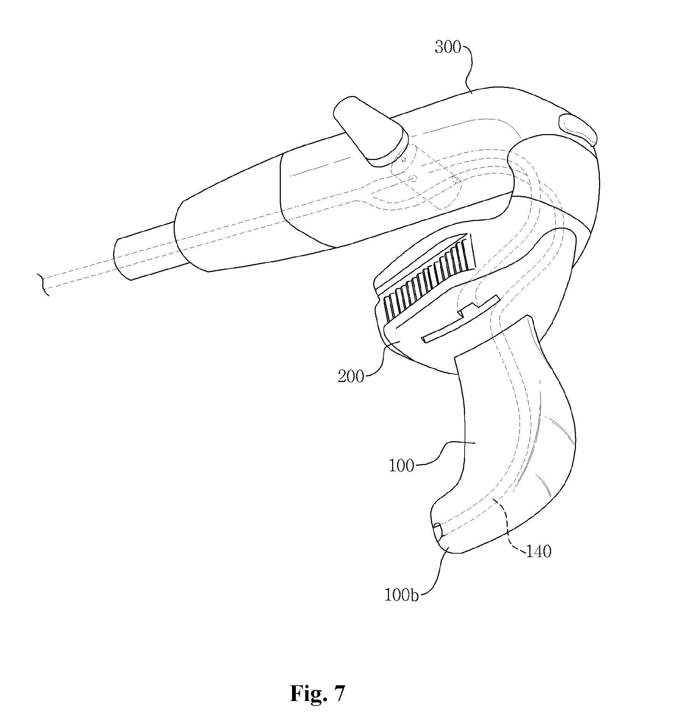

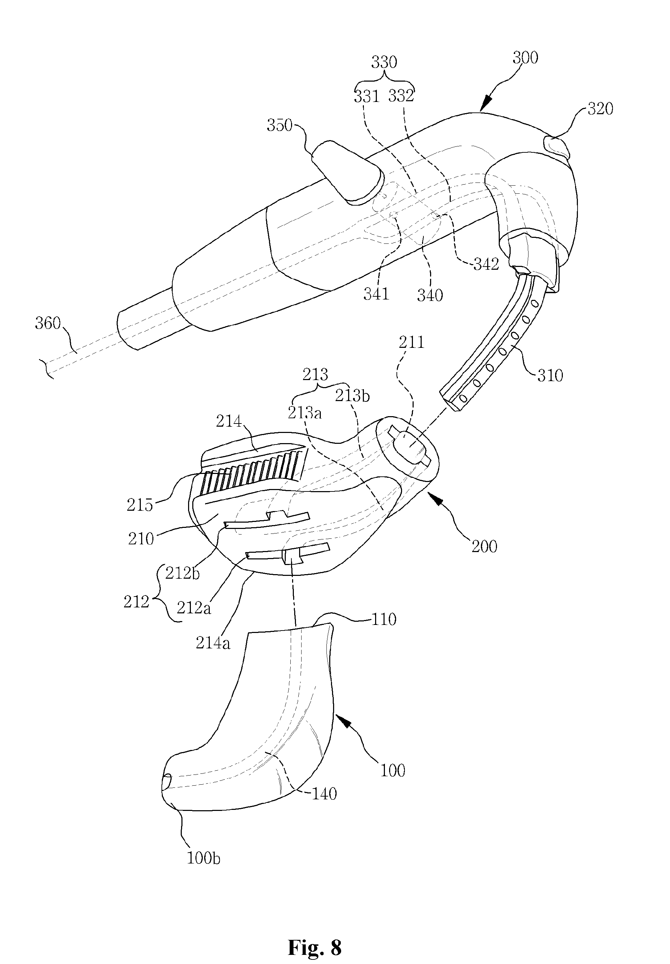

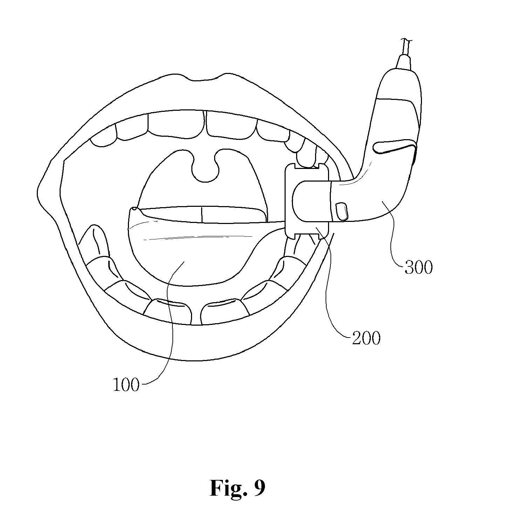

FIG. 7 is a view illustrating an embodiment of an intraoral illumination device according to the present invention. FIG. 8 is an exploded perspective view of the intraoral illumination device according to the present invention. FIG. 9 is a view showing the installation of the intraoral illumination device of the present invention in the mouth of the patient.

Referring to the drawings, the intraoral illumination device according to the present invention includes a handpiece 300, a bite block 200 and a TIPS 100.

The handpiece 300 includes a light source 310 on a first end thereof. The light source 310 is inserted into the bite block 200 which will be explained in detail later. The light source 310 directly illuminates the mouth cavity of the patient.

Furthermore, the handpiece 300 has, on a portion of the outer surface thereof, a light source control switch 320 which turns on/off the light source 310.

In the embodiment, a touch switch is used as the light source control switch 320, so that when the user touches the switch with his/her hand, the light source 310 is turned on, and when the user touches the switch once more, the light source 310 is turned off.

Of course, in addition to the touch switch, various well known switches may be used as the light source control switch 320.

In the embodiment, the light source 310 may have a plurality of LEDs and comprise a bar-shaped surface light source to facilitate insertion thereof into the bite block 200 and minimize formation of a shadow while illuminating the mouth cavity.

In addition, the intraoral illumination device may further include an ultraviolet lamp (not shown) which is provided on the light source 310 at a portion adjacent to the LEDs and emits ultraviolet rays into the mouth to sterilize the interior of the mouth when dental treatment is performed.

The ultraviolet lamp may be disposed at any position on the handpiece 300, as long as it can apply ultraviolet rays to the mouth cavity.

Furthermore, the intraoral illumination device may further include a camera (not shown) which is provided on the light source 310 to record images of the mouth as it is being treated.

Therefore, the present invention enables the doctor or patient to observe images of the treatment and thus further enhance the reliability of the treatment.

Of course, the camera may be disposed at any position on the handpiece 300, so long as it can photograph the interior of the mouth. In the present invention, the camera is provided on the light source 310 to effectively obtain images of the treatment.

A suction tube 330 is formed through the handpiece 300 and extends from the first end of the handpiece 300 to the second end thereof to transfer saliva to an external suction device (not shown).

Furthermore, a valve 340 which opens or closes the suction tube 300 is provided in the handpiece 300. A valve control lever 350 is provided on the outside surface of the handpiece 300 to control the operation of the valve 340.

In detail, the suction tube 330 comprises two suction tubes including a first suction tube 331 and a second suction tube 332 which are separated from each other. The valve 340 is provided on the both two suction tubes 331 and 332. In other words, the valve 340 comprises a single valve which selectively opens or closes the suction tubes 331 and 332.

The valve 340 has a first valve hole 341 and a second valve hole 342 which are formed in different directions and are respectively connected to the first suction tube 331 and the second suction tube 332 to selectively open the first suction tube 331 and the second suction tube 332.

In detail, the first and second suction tubes 331 and 332 can be selectively opened or closed by controlling the valve control lever 350 in such a way that when the first suction tube 331 is opened by opening the first valve hole 341, the second suction tube 332 is closed, and when the second suction tube 332 is opened by opening the second valve hole 342, the first suction tube 331 is closed.

Furthermore, the valve 340 may comprise two valves which are respectively provided on the first suction tube 331 and the second suction tube 332. The valve control lever 350 may also comprise two valve control levers which respectively control the two valves.

The suction tubes 331 and 332 join together between the valve 340 and the external suction device (not shown) so that the external suction device can efficiently suck saliva.

Of course, the suction tubes 331 and 332 may be directly connected to the external suction device (not shown).

The bite block 200 is placed between the upper and lower teeth which are positioned at the first side in the mouth and bitten to open the mouth. The bite block 200 includes a block body 210 which is substantially bitten between the upper and lower teeth.

A light source insert hole 211 is formed in a first end of the block body 210 so that the light source 310 is inserted into the light source insert hole 211.

In detail, the light source insert hole 211 is formed in the side of the block body 210 that is exposed out of the mouth when the patient bites the block body 210.

Also, the block body 210 is made of transparent material having high light transmissivity so that light emitted from the light source 310 inserted into the light source insert hole 211 can be effectively transmitted out of the block body 210

Furthermore, a TIPS coupling hole 212 to which the TIPS 100 is coupled is formed in a side of the block body 210 which faces the center of the mouth cavity when the patient bites the block body 210.

A saliva transfer tube 213 is formed in the block body 210 and extends from the first end of the block body 210 to the TIPS coupling hole 212. The saliva transfer tube 213 is spaced apart from the light source insert hole 211 by a predetermined distance on the first end of the block body 210.

The TIPS coupling hole 212 comprises a plurality of TIPS coupling holes 212a and 212b which are spaced apart from each other. In the embodiment, the TIPS coupling hole 212 comprises two TIPS coupling holes 212a and 212b, that is, a first TIPS coupling hole 212a and a second TIPS coupling hole 212b, to which a left side TIPS and a right side TIPS can be respectively coupled.

The saliva transfer tube 213 may comprise two saliva transfer tubes 213a and 213b which are spaced apart from each other and extend from the first end of the block body 210 to the respective TIPS coupling holes 212a and 212b.

Alternatively, the saliva transfer tube 213 may comprise a single saliva transfer tube which branches out into two parts which are respectively connected to the TIPS coupling holes 212a and 212b.

First ends of the saliva transfer tubes 213a and 213b are respectively connected to the suction tubes 331 and 332 of the handpiece 300. A second end of one of the saliva transfer tubes 213a and 213b is connected to the saliva suction tube 140 of the TIPS 100.

Saliva 1 collected in the mouth is sucked into the saliva suction tube 140 of the TIPS 100, and transferred to the suction tube 330 of the handpiece 300 via the saliva transfer tube 213 of the bite block 200, and then drawn into the external suction device (not shown).

In more detail, the suction tube 330 is connected to the external suction device (not shown) through a saliva discharge tube 360, so that saliva 1 transferred to the suction tube 330 is drawn into the external suction device (not shown) through the saliva discharge tube 360.

Furthermore, the saliva discharge tube 360 and the suction tube 330 may be integrally formed into a single tube.

Meanwhile, bite depressions 214 are formed in upper and lower portions of the block body 210 which come into close contact with the upper and lower teeth of the patient. An anti-slip uneven surface 215 is formed in the bottom of each bite depression 214 to prevent the block body 210 from slipping relative to the upper or lower teeth of the patient.

Therefore, the block body 210 is prevented from undesirably moving in any direction when the block body 210 is in a state of being bitten between the upper and lower teeth of the patient. As a result, the light source 310 can stably illuminate the mouth cavity.

The TIPS 100 of this embodiment has the same structure as that of the TIPS 100 illustrated with reference to FIGS. 4 through 6, therefore further explanation will be omitted.

Although the preferred embodiments of the present invention have been disclosed for illustrative purposes, those skilled in the art will appreciate that various modifications, additions and substitutions are possible, without departing from the scope and spirit of the invention.

### [Industrial Applicability]

In the medical field, particularly, the dental or otorhinolaryngological field, a TIPS (twist isolated plate of suction), a bite block and an intraoral illumination device having a saliva suction function according to the present invention can be effectively used for performing medical treatment in the mouth of a patient.

## Claims

1. A TIPS (twist isolated plate of suction) for protecting a tongue of a patient when a dental treatment is performed, the TIPS being removably coupled to a bite block bitten between upper and lower teeth in a first side of a mouth of the patient to open the mouth of the patient, the TIPS having:
a first side coupled to the bite block; and
a second side covering a first side surface of the tongue and extending towards a second-side tongue root in the mouth.

2. The TIPS according to claim 1, comprising:
a block coupling part coupled to the bite block;
a TIPS body extending from the block coupling part and being curved towards the second-side tongue root; and
a tongue protection part extending downwards from an edge of the TIPS body to cover the first side surface of the tongue.

3. The TIPS according to claim 2, wherein the tongue protection part covers a tip of the tongue and isolates the tongue from teeth positioned in a second side of the mouth of the patient.

4. The TIPS according to any one of claims 1 through 3, wherein the second side of the TIPS extends from the first side of the TIPS along an imaginary extension axis,
the second side of the TIPS being twisted around the imaginary extension axis at a predetermined angle to surround the first side surface of the tongue.

5. The TIPS according to any one of claims 1 through 3, wherein a saliva suction tube is formed through the first side of the TIPS and the second side of the TIPS to suck saliva collected between a throat of the patient and the second-side tongue root.

6. The TIPS according to claim 5, wherein the saliva suction tube comprises a plurality of saliva suction tubes.

7. The TIPS according to claim 4, wherein a saliva suction tube is formed through the first side of the TIPS and the second side of the TIPS to suck saliva collected between a throat of the patient and the second-side tongue root.

8. The TIPS according to claim 7, wherein the saliva suction tube comprises a plurality of saliva suction tubes.

9. A bite block, comprising:
a block body bitten between upper and lower teeth in a first side of a mouth of a patient to open a mouth of the patient;
a light source insert hole formed in a first side of the block body so that an external light source is inserted into the light source insert hole;
a TIPS coupling hole formed in the block body at a position facing a center of the mouth so that a TIPS (twist isolated plate of suction) is coupled to the TIPS coupling hole; and
a saliva transfer tube formed through the first side of the block body at a position spaced apart from the light source, the saliva transfer tube communicating with the TIPS coupling hole and being connected to an external suction tube so that saliva drawn into the TIPS coupling hole is transferred to the external suction tube through the saliva transfer tube.

10. The bite block according to claim 9, wherein
the TIPS coupling hole comprises a plurality of TIPS coupling holes spaced apart from each other, and
the saliva transfer tube comprises a plurality of saliva transfer tubes spaced apart from each other, the saliva transfer tubes respectively communicating with the TIPS coupling holes.

11. The bite block according to claim 10, wherein
the TIPS coupling holes comprise two TIPS coupling holes including a first TIPS coupling hole and a second TIPS coupling hole, and
the saliva transfer tubes comprise a first saliva transfer tube and a second saliva transfer tube which are respectively connected to the first TIPS coupling hole and the second TIPS coupling hole.

12. The bite block according to claim 9, wherein
the TIPS coupling hole comprises a plurality of TIPS coupling holes spaced apart from each other, and
the saliva transfer tube branches out into two parts connected to the respective TIPS coupling holes.

13. The bite block according to claim 12, wherein the TIPS coupling holes comprise two TIPS coupling holes including a first TIPS coupling hole and a second TIPS coupling hole which are spaced apart from each other.

14. The bite block according to any one of claims 9 through 13, wherein bite depressions are respectively formed in upper and lower portions of the block body which are bitten between the upper and lower teeth of the patient.

15. The bite block according to claim 14, wherein an anti-slip uneven surface is formed in each of the bite depressions to prevent the block body from slipping relative to the upper or lower teeth of the patient.

16. The bite block according to claim 15, wherein the anti-slip uneven surface is formed in a bottom of the bite depression.

17. The bite block according to claim 9, wherein the block body comprises a transparent block body allowing light emitted from the light source to be transmitted through the block body.

18. An intraoral illumination device, comprising:
a handpiece comprising a light source provided on a first end thereof;
a bite block coupled to the first end of the handpiece, the bite block transmitting light of the light source into a mouth of a patient and being bitten between upper and lower teeth in a first side of the mouth to open the mouth; and
a TIPS (twist isolated plate of suction) for protecting a tongue of the patient, the TIPS having a first side coupled to the bite block, and a second side covering a first side surface of the tongue of the patient and extending towards a second-side tongue root in the mouth.

19. The intraoral illumination device according to claim 18, wherein the TIPS comprises:
a block coupling part coupled to the bite block;
a TIPS body extending from the block coupling part and being curved towards the second-side tongue root; and
a tongue protection part extending downwards from an edge of the TIPS body to cover the first side surface of the tongue.

20. The intraoral illumination device according to claim 19, wherein the tongue protection part covers a tip of the tongue and isolates the tongue from teeth positioned in a second side of the mouth of the patient.

21. The intraoral illumination device according to any one of claims 18 through 20, wherein the second side of the TIPS extends from the first side of the TIPS along an imaginary extension axis,
the second side of the TIPS being twisted around the imaginary extension axis at a predetermined angle to surround the first side surface of the tongue.

22. The intraoral illumination device according to any one of claims 18 through 20, wherein a saliva suction tube is formed through the first side of the TIPS and the second side of the TIPS to suck saliva collected between a throat of the patient and the second-side tongue root.

23. The intraoral illumination device according to claim 22, wherein the saliva suction tube comprises a plurality of saliva suction tubes.

24. The intraoral illumination device according to claim 21, wherein a saliva suction tube is formed through the first side of the TIPS and the second side of the TIPS to suck saliva collected between a throat of the patient and the second-side tongue root.

25. The intraoral illumination device according to claim 24, wherein the saliva suction tube comprises a plurality of saliva suction tubes.

26. The intraoral illumination device according to claim 22, wherein the bite block comprises:
a block body bitten between the upper and lower teeth in the first side of the mouth of the patient;
a light source insert hole formed in a first side of the block body so that the light source is inserted into the light source insert hole; and
a TIPS coupling hole formed in the block body at a position facing a center of the mouth so that the TIPS is coupled to the TIPS coupling hole.

27. The intraoral illumination device according to claim 26, wherein the handpiece further comprises a suction tube provided through the first end and a second of the handpiece, the suction tube being connected to an external suction device, and
the bite block further comprises a saliva transfer tube connected to the suction tube, the saliva transfer tube communicating with the TIPS coupling hole so that saliva drawn into the TIPS coupling hole is transferred to the suction tube through the saliva transfer tube.

28. The intraoral illumination device according to claim 27, wherein
the TIPS coupling hole comprises a plurality of TIPS coupling holes spaced apart from each other, and
the saliva transfer tube comprises a plurality of saliva transfer tubes spaced apart from each other, the saliva transfer tubes respectively communicating with the TIPS coupling holes.

29. The intraoral illumination device according to claim 28, wherein
the suction tube comprises a plurality of suction tubes, and
the saliva transfer tubes are respectively connected to the suction tubes.

30. The intraoral illumination device according to claim 29, wherein
the suction tubes comprise two suction tubes including a first suction tube and a second suction tube,
the TIPS coupling holes comprise two TIPS coupling holes including a first TIPS coupling hole and a second TIPS coupling hole, and
the saliva transfer tubes comprise a first saliva transfer tube and a second saliva transfer tube which are respectively connected to the first suction tube and the second suction tube, the first and second saliva transfer tubes respectively communicating with the first TIPS coupling hole and the second TIPS coupling hole.

31. The intraoral illumination device according to claim 27, wherein
the TIPS coupling hole comprises a plurality of TIPS coupling holes spaced apart from each other, and
the saliva transfer tube branches out into a plurality of parts communicating with the respective TIPS coupling holes.

32. The intraoral illumination device according to claim 30, wherein bite depressions are respectively formed in upper and lower portions of the block body which are bitten between the upper and lower teeth of the patient.

33. The intraoral illumination device according to claim 32, wherein an anti-slip uneven surface is formed in each of the bite depressions to prevent the block body from slipping relative to the upper or lower teeth of the patient.

34. The intraoral illumination device according to claim 33, wherein the anti-slip uneven surface is formed in a bottom of the bite depression.

35. The intraoral illumination device according to claim 30, wherein the handpiece comprises a valve to open or close the suction tubes.

36. The intraoral illumination device according to claim 35, wherein the valve comprises a single valve provided on the suction tubes in the handpiece, the valve selectively opening or closing the suction valve.

37. The intraoral illumination device according to claim 36, wherein the handpiece further comprises a valve control lever to control operation of the valve.

38. he intraoral illumination device according to claim 37, wherein the handpiece further comprises a light source control switch provided on an outer surface of the handpiece to turn on/off the light source.

39. The intraoral illumination device according to claim 38, wherein the light source control switch comprises a touch switch.

40. The intraoral illumination device according to claim 18, wherein the bite block comprises a transparent bite block allowing light emitted from the light source to transmit through the bite block body.

41. The intraoral illumination device according to claim 18, wherein the light source comprises an LED.

42. The intraoral illumination device according to any one of claims 18 through 20, further comprising:
an ultraviolet lamp provided at a predetermined position on the handpiece to apply ultraviolet rays to an interior of the mouth.

43. The intraoral illumination device according to claim 42, wherein the ultraviolet lamp is provided on the light source.

44. The intraoral illumination device according to any one of claims 18 through 20, further comprising:
a camera provided at a predetermined position on the handpiece to record images of the mouth when treatment is performed.

45. The intraoral illumination device according to claim 44, wherein the camera is provided on the light source.
